# EUROPEAN PATENT APPLICATION

(11) **EP 4 091 533 A1**
(43) Date of publication of application: **23.11.2022**
(21) Application number: 22174420.4
(22) Date of filing: 19.05.2022
(51) Int. Cl.: A61B 3/00, A61B 3/032, A61B 3/04, G02B 3/14, G02B 27/01, G02C 7/02

(54) **METHOD, COMPUTER PROGRAM, AND SYSTEM FOR DETERMINING AT LEAST ONE OPTICAL PROPERTY OF A VISUAL AID FOR A PERSON**

(30) Priority: 19.05.2021 DE 102021113026
(71) Applicant: Optotune AG, 8953 Dietikon (CH)
(72) Inventor: ASCHWANDEN, Manuel, 6319 Allenwinden (CH)
(74) Representative: Schulz Junghans Patentanwälte PartGmbB

(57) **Abstract**

The present invention relates to a method for determining at least one optical property of a visual aid (e.g. eyeglasses) for a person, the method comprising the steps of: Observation of a test pattern (T) by the person through an tunable lens (20, 21) of a virtual reality (VR) and/or augmented reality (AR) headset (2) worn by the person, the tunable lens (20, 21) being positioned in front of an eye of the person, and adjusting at least one optical property of the tunable lens (20, 21) so that the person perceives the test pattern (T) as being sharp, or adjusting at least one optical property of the tunable lens (20, 21) until a sensor device (30, 31) detects accommodation of the eye of the person to the test pattern (T).

## Description

### Specification

The present invention relates to a method and a system for determining at least one optical property of a visual aid (e.g. eyeglasses) for a person.

Regarding the measurement of eyeglass prescriptions, it is desirable to perform such measurement in an easy and effortless manner that does not require a person to visit a certain location, particularly regarding generation of the eyeglass prescription as well as regarding acquiring eyeglasses made according to the prescription.

So far, such measurements are e.g. conducted by a physician or optician using a phoropter and trial lenses. Furthermore, devices such as manual flip mounts and light sources can be used for measuring the desired optical properties. Particularly changing a distance of the eye to an optical target is frequently employed.

Furthermore, augmented reality (AR) headsets are known in the state of the art that are configured to overlay augmented content, such as 3D content, 2D overlays, text, virtual objects, etc., onto a user's view of the surrounding real-world environment. For example, an AR headset is often configured to show a view of the real world that has been augmented to include either or both static and dynamic 2D or 3D content. On the other hand, a virtual reality (VR) headset is configured to present the user with a completely virtual 2D or 3D environment that replaces the user's view of the surrounding real-world environment. It is also possible to have a headset that is configured to present AR and/or VR.

Based on the above, the problem to be solved by the present invention is to provide a method, a computer program, and a system for determining at least one optical property of an eyeglass prescription that is more convenient for the person and easy to use.

This problem is solved by a method having the features of claim 1, a computer program having the features of claim 12, and a system having the features of claim 13.

Preferred embodiments of these aspects of the present invention are stated in the corresponding sub claims and are described below.

According to claim 1, a method for adapting a visual aid for a person, the method comprising the steps of:
a) providing a virtual reality (VR) and/or augmented reality (AR) headset comprising a tunable lens,
b) Observation of a test pattern (can be every image appropriate for testing eyesight of a person) by the person through the tunable lens, and
c) Adjusting an optical property of the tunable lens to an optimal value, wherein an image of the test pattern perceived by the person is changed by adjusting said optical property.

Particularly, virtual reality (VR) and/or augmented reality (AR) headset means that the headset is configured to present to the person a virtual reality (VR) and/or an augmented reality (AR), see also above. In each embodiment of the present invention a headset can be used that is configured to present VR (i.e. a VR headset), or a headset can be used that is configured to present AR (i.e. an AR headset), or a headset can be used that is configured to present both virtual reality (VR) and augmented reality (AR), e.g. at the same time and/or in different operating modes of the headset.

Particularly, the VR and/or AR headset used in the framework of the present invention can comprise two tunable lenses, a holding structure configured to hold each tunable lens in front of an eye of the person in a defined distance to the respective eye (particularly, the holding structure can comprise at least one headband or temples). The headset can further comprise two displays each display being configured to be positioned in front of an eye of the person. Furthermore, the headset can comprise a shutter for each eye, particularly for testing prism power and/or prism angle, the respective shutter being configured to exempt the respective eye from viewing a scene.

The invention thus allows a precise and cost-effective measurement of optical properties, particularly of all optical properties needed for a visual aid (particularly for an eyeglass prescription) and particularly other optometric parameters, preferably at home.

Furthermore, the method can also be employed for efficient eye training.

Furthermore, the method can also be used to study the ability of accommodation and speed of accommodation (changing of optical power). The young human eye can change focus from infinity to approximately 15 diopters due to a change in ciliary muscle contraction which reduces with age. The muscle flexibility can be investigated.

Furthermore, the eye's light sensitivity can be determined.

Furthermore, the method can also be used to identify injuries of the eye. For example, to infer after a stroke on how far the eye can move and see pictures (measurement of field of view of the eye).

Furthermore, according to an embodiment of the method, the VR and/or AR headset is configured to display the test pattern via at least one display of the VR and/or AR headset. However, the test pattern can also be a physical test pattern observed through the VR and/or AR headset or a test pattern displayed in the first place by a separate display (i.e. a display not comprised by the VR and/or AR headset).

According to a further embodiment of the method, step c) further comprises that
- the person adjusts the optical property of the tunable lens to an optimal value upon perceiving said image, or
- upon adjusting the optical property, a sensor device detects an optimal value of the optical property to which the optical property is adjusted.

Furthermore, according to an embodiment, the sensor device can comprise a camera, particularly two cameras, configured to monitor the person's eye. In an embodiment, the sensor device comprises two cameras (i.e. one camera per eye of the person), each camera configured to observe an associated eye of person. Accommodation of the eye (e.g. in case the optical power is sphere power) can be detected by adjusting the optical property in a continuous fashion (or in small successive increments) while observing the eye of the person with the sensor device, particularly with said camera(s), wherein the test pattern is considered to be perceived as a sharp image by the person in case the lens of the eye of the person stops changing its form at a certain value of the optical property. This can be inferred by automatically analyzing images of the lens of the eye acquired with the sensor device, particularly with said camera(s).

Furthermore, according to an embodiment of the method according to the present invention, adjusting the optical property comprises activating an actuator to cause the actuator to change a shape of a surface of the tunable lens. Particularly, the actuator can be actuated by the person, e.g. manually or by voice command. Alternatively, the actuator can be actuated automatically, e.g. when used in conjunction with the sensor device.

Further, according to an embodiment, the optical property of the lens is one of: sphere power, cylinder power, cylinder angle, prism power, prism angle. Particularly, according to an embodiment any combination of these optical properties of the tunable lens can be adjusted. Particularly, all of these optical properties are adjusted and particularly comprised by an eyeglass prescription generated by way of the method according to the invention, which will be described in more detail below.

Here and in the following, meridians of the tunable lens describe imaginary straight lines extending through the center of the circumcircle, wherein different meridians extend at an angle with respect to each other.

Sphere (abbreviated as SPH) power indicates the amount of lens power, measured in diopters of focal length. The deflection of the membrane for sphere is equal in all meridians of the tunable lens. The tunable lens is arranged to alter the lens power by a definable deformation of the membrane.

Cylinder (abbreviated as CYL) power indicates the lens power for astigmatism of the tunable lens. The membrane has a non-spherical surface shape for generating cylinder power. In particular, for generating cylinder power the membrane has a shape so that along a first meridian the membrane has no added curvature, and along a second meridian the membrane has the maximum added curvature, wherein the first meridian and the second meridian extend perpendicular with respect to each other. The tunable lens is arranged to alter the curvature of the membrane along the second meridian.

Cylinder axis describes the angle of the first meridian, which has no added curvature to correct astigmatism. In other words, the cylinder axis is the angle of the first lens meridian that is 90 degrees away from the second meridian, wherein the second meridian contains the cylinder power. The cylinder axis is defined with an angle from 1° to 180°. The tunable lens may be arranged to alter the cylinder axis from 1° to 180°angle.

In particular, optical properties are prism power and prism axis and add. Prism power is the amount of prismatic power of the tunable lens, measured in prism diopters ("p.d." or a superscript triangle). Prism power is indicated in either metric or fractional English units (0.5 or ½, for example). Prism corresponds to a tilt of the membrane's surface with respect to the optical axis. Prism power defines absolute of the angle by which the membrane's surface is tilted. The tunable lens may be arranged to alter the prism power.

Prism axis is the direction of prismatic power of the tunable lens. The prism axis indicates the angle of the meridian around which the surface of the tunable lens is tilted with respect to the optical axis. The prism axis may extend along any meridian. The prism axis may be defined by an angle from 1° to 360°. The tunable lens may be arranged to alter the prism axis from 1° to 360°.

Add is the added magnifying power applied to a portion of the tunable lens. In particular, a tunable lens with Add is a multifocal lens. The added magnifying power may range from +0.75 to +3.00 diopters.

According to a further embodiment of the method, the at least one optical property is sphere power.

In this regard, according to a further embodiment, step c) further comprises
adjusting (as a further optical property) the cylinder angle of the tunable lens with the tunable lens comprising a predefined cylinder power so that the visual perception of the test pattern by the person is optimized;
or
- adjusting (as a further optical property) the cylinder angle of the tunable lens with the tunable lens comprising a predefined cylinder power until the sensor device detects accommodation of the eye of the person to the test pattern.

Particularly, cylinder angle is an optical property relevant for correcting astigmatism.

Astigmatism is also perceived as blur. However, the blur is not homogeneous across the field of view. It is a blur gradient. In astigmatism, the area of maximum blur typically extends linearly across the field of view. Astigmatism can also be perceived as a curvature of the image. That is, even when adjusting cylinder (angle or power), the lens of the eye will change and try to find a sharp point. The system tries to adjust the tunable lens so that the lens of the eye is no longer looking for a sharp point. The lens in the eye will therefore no longer change because it is no longer looking for a sharp point. This can be detected with the sensor device, whereby the optimum value can be detected.

Furthermore, according to an embodiment, step c) further comprises
- adjusting (as a further optical property) the cylinder power of the tunable lens to an optimal value so that the visual perception of the test pattern by the person is optimized, wherein upon adjusting, the tunable lens comprises the optimal value of the cylinder angle
   or
- adjusting (as a further optical property) the cylinder power of the tunable lens until the sensor device detects accommodation of the eye of the person to the test pattern, wherein upon adjusting, the tunable lens comprises the optimal value of the cylinder angle, and wherein the cylinder power of the tunable lens at which accommodation is detected, corresponds to the optimal value of the cylinder power.

Here, detecting accommodation can be carried out as explained in conjunction with the cylinder angle above.

Further, according to yet another embodiment, step c) further comprises
- adjusting (as a further optical property) the prism angle of the tunable lens to an optimal value so that the visual perception of the test pattern by the person is optimized, wherein upon adjusting, the tunable lens comprises a predefined prism power
   or
- adjusting (as a further optical property) the prism angle of the tunable lens until the sensor device detects accommodation of the eye of the person to the test pattern, wherein upon adjusting, the tunable lens comprises a predefined prism power, and wherein the prism angle of the tunable lens at which accommodation is detected, corresponds to the optimal value of the prism angle.

Particularly in case of prism angle, the movement of the pupil of the eyes is monitored while a beam path to the eyes is alternately opened and closed (e.g. by means of the shutters of the headset). Particularly, the test pattern is presented at a fixed position, which the person can perceive alternately with the right eye and the left eye. Prism angle is adjusted until, when alternately opening the shutters in front of the eyes, the respective pupil no longer needs to track to see the target. In other words, adjusting prism angle allows correcting vision so that both eyes see in the same direction without being aligned in the same direction.

Further, according to an embodiment, step c) further comprises
- adjusting (as a further optical property) the prism power of the tunable lens so that the visual perception of the test pattern by the person is optimized, wherein upon adjusting, the tunable lens comprises the optimal value of the prism angle,
   or
- adjusting (as a further optical property) the prism power of the tunable lens until the sensor device detects accommodation of the eye of the person to the test pattern, wherein upon adjusting, the tunable lens comprises the optimal value of the prism angle, and wherein the prism power of the tunable lens at which accommodation is detected, corresponds to the optimal value of the prism power.

Here, regarding detection of accommodation of the eyes with respect to prism power, the same method used for prism angle can be applied.

According to a further preferred embodiment of the method according to the present invention, the tunable lens comprises a shaping element for changing a shape of a surface of the tunable lens and to thereby change the at least one optical property (particularly of each of the above-described optical properties or of a selection thereof).

Particularly, according to an embodiment, the shaping element comprises a non-circular contour defined by an inner edge of the shaping element. Furthermore, particularly, the shaping element is connected to a membrane of the tunable lens and circumvents an optically active area of the membrane, which area forms said surface of the tunable lens.

Furthermore, particularly, the shaping element can be a deformable shaping element (so-called soft shaper), wherein the tunable lens comprises an actuator that is configured to deform the shaping element and therewith said surface to cause the tunable lens to have a defined sphere power, cylinder power, cylinder angle, prism power and prism angle. Each of these optical properties can be adjusted individually by deforming the shaping element using the actuator.

Particularly, the actuator can be configured to allow adjusting of the sphere power of the tunable lens in 0.25 diopter steps or other suitable increments.

Further, in an embodiment, the further comprises the step of
d) fabricating the visual aid, the visual aid comprising the optimal value of the respective optical property (e.g. one, several or all of: sphere power, cylinder angle, cylinder power, prism angle, prism power).

According to yet another preferred embodiment of the method according to the present invention, the method further comprises the step of
d) transmitting the optimal value of the respective optical property (e.g. in form of an eyeglass prescription) to a manufacturer for manufacturing the visual aid or to a manufacturing device for optical elements (such as lenses of eyeglasses), and
e) fabricating optical elements having the optimal value of the respective optical property, and particularly integrating the optical elements in a visual aid, particularly eyeglasses

Particularly, the eyeglass prescription can comprise each of the above-described optimal values of the optical properties or a selection thereof.

Furthermore, according a preferred embodiment of the method, transmitting said optimal values (e.g. in form of an eyeglass prescription) comprises transmitting the optimal values of the respective optical property via a computer network.

Preferably, the computer network uses TCP/IP for transmitting the eyeglass prescription. Preferably, said computer network is the internet.

Particularly, the eyeglass prescription is transmitted from a computer to which the VR and/or AR headset is connected (e.g. as a user interface) to the manufacturer via the computer network (e.g. via an e-mail or an upload, e.g. via a website of the manufacturer)

Furthermore, the method according to the present invention, the method can further comprise the step of long-term monitoring of at least one property of the eye (or the eyes) of the person. Particularly, the user may monitor the respective eye repeatedly (e.g. on a regular basis), which enables to track the respective property of the respective user's eye over a long period of time, preferably at least a weak, or at least a month, or at least a year.

According to a further embodiment of the method, the method comprises the step of measuring a speed of an accommodation of the eye (e.g. left and/or right eye) of the person.

According to a further embodiment of the method, the method comprises the step of measuring a speed of a movement of the eye (e.g. left and/or right eye) of the person.

Furthermore, the method according to the present invention can be used to detect a cataract of the person.

According to yet another embodiment, the method of the present invention comprises the step of conducting a training of the eye (particularly of the left and/or right eye) of the person, which training can comprise tuning the tunable lens through different values of sphere power and let the user accommodate the respective eye (e.g. left and/or right eye) accordingly.

According to yet another embodiment, the method comprises the step of providing a relaxation of the eye of the person (e.g. of the left and/or right eye), which relaxation can comprise tuning the focal (i.e. sphere) power of the tunable lens to move the eye of the person.

In the above, only one tunable lens has been described in conjunction with the method, system, and computer program.

However, the VR and/or AR headset can comprise a tunable lens in front of each eye of the person. Thus, the VR and/or AR headset can comprise a first tunable lens for the left eye and a second tunable lens for the right eye. Each of these tunable lenses can be formed according to embodiments described herein with regard to the tunable lens.

The above-described adjustments of the various optical properties can be performed for one eye (e.g. left or right eye) and thereafter for the other eye of the person. This can involve covering that eye whose associated tunable lens is currently not being adjusted. For covering the respective eye, the VR and/or AR headset can comprise a shutter. Particularly, in case prism angle and prism power is adjusted, these optical properties are adjusted for both eyes in an alternating fashion.

A computer program comprising instructions which, when the computer program is executed on a computer cause the computer to conduct the method according to one of the preceding claims.

Particularly, the VR and/or AR headset can be connected to the computer. Furthermore, the computer can also be integrated into the VR and/or AR headset.

A system for determining at least one optical property of a visual aid (e.g. eyeglasses) for a person, the system comprising a VR and/or AR headset configured to be worn by a person, the VR and/or AR headset comprising a tunable lens configured to be positioned in front of an eye of the person when the VR and/or headset is worn by the person, wherein the tunable lens is adjustable to adjust at least one optical property of the tunable lens, and wherein the system comprises an input device configured to be operated by the person to adjust the tunable lens and therewith the optical property, and/or wherein the system comprises a sensor device, wherein the sensor device is configured to detect accommodation of the said eye to a target pattern observed through the tunable lens and to provide data indicative of the detected accommodation, wherein the system is configured to control adjusting of the tunable lens based on said data. Furthermore, according to an embodiment of the system, the sensor device can comprise a camera, particularly two cameras, configured to monitor the person's eye (e.g. left and/or right eye). In an embodiment, the sensor device of the system comprises two cameras (i.e. one camera per eye of the person), each camera configured to observe an associated eye of person.

Particularly, for detecting accommodation of the eye, the system is configured to adjust the optical property (e.g. in a continuous fashion or in small successive increments) while monitoring the respective eye of the person by the camera(s) of the sensor device, wherein the test pattern is considered to be perceived as a sharp image by the person in case the lens of the eye of the person stops changing its form at a certain value of the optical property being adjusted.

According to a further embodiment of the system according to the present invention, the system comprises at least one display, the display configured to display the test pattern, wherein particularly the at least one display is comprised by the VR and/or AR headset.

Preferably, the system, particularly the VR and/or AR headset comprises an actuator configured to change a shape of a surface of the tunable lens to adjust said at least one optical property. In case the VR and/or AR headset comprises two tunable lenses, the system (particularly VR and/or AR headset) comprises two actuators, one for each tunable lens.

According to an embodiment of the system, said at least one optical property is one of: sphere power, cylinder power, cylinder angle, prism power, prism angle.

According to a preferred embodiment, the respective tunable lens comprises a shaping element for changing the shape of the surface of the tunable lens in order to adjust the optical property in question (e.g. one of sphere power, cylinder power, cylinder angle, prism power, prism angle, or a combination thereof).

Particularly, according to an embodiment of the system, the shaping element comprises a non-circular inner edge defining a contour. Furthermore, particularly, the shaping element is connected to a membrane of the tunable lens and circumvents an optically active area of the membrane, which area forms said surface of the tunable lens. Furthermore, particularly, the shaping element can be a deformable shaping element (see also above).

Particularly, the tunable lens comprises an actuator that is configured to deform the shaping element and therewith said surface to cause the tunable lens to have a defined sphere power, cylinder power, cylinder angle, prism power and prism angle. Each of these optical properties can be adjusted individually by deforming the shaping element using the actuator.

Furthermore, according on embodiment, the system comprises a computer, particularly operatively connected to the VR and/or AR headset and particularly configured to derive an eyeglass prescription comprising the at least one optical property (or all of the afore-mentioned optical properties or a selection thereof, which means every possible combination). The computer can for instance be one of: a desktop computer, a laptop, a tablet computer, a mobile phone (e.g. smart phone).

Particularly, according to an embodiment, the computer is configured to transmit the respective optical property or eyeglass prescription to a remote server via a computer network.

Preferably, in an embodiment, the computer network uses TCP/IP for transmitting the respective optical property or eyeglass prescription. Preferably, said computer network is the internet.

In the above, in connection with some embodiments, only one tunable lens has been described in conjunction with the method, system, and computer program.

However, regarding the method, computer program and system according to the present invention the VR and/or AR headset can comprise a tunable lens in front of each eye of the person. Thus, the VR and/or AR headset can comprise a first tunable lens for the left eye and a second tunable lens for the right eye. Each of these tunable lenses can be formed according to embodiments described herein with regard to the tunable lens. Also, the system, particularly the VR and/or AR headset, can comprise an actuator according to any embodiment described herein for each tunable lens.

The above-described adjustments of the various optical properties can be performed for one eye (e.g. left or right eye) and thereafter for the other eye of the person. Particularly cylinder or sphere are measured for one eye at a time. When prism power or angle is tested both eyes are preferably considered as these quantities correspond to a relative quantity between the two eyes, respectively. This can involve covering that eye whose associated tunable lens is currently not being adjusted. For covering the respective eye, the VR and/or AR headset can comprise a shutter.

Furthermore, according to a preferred embodiment of the invention, the tunable lens, can adjust not only sphere, but also cylinder and prism via a single membrane, and thus the three most important parameters can be elicited in case of defective vision.

Furthermore, according to an embodiment of the invention, a preferred procedure for determining the optical parameters comprises a sequence of adjustment of the parameters, preferably in the order: sphere, optical power, astigmatism, prism.

Furthermore, according to a preferred embodiment of the invention, an active intervention of the user takes place by selecting the parameters (e.g. sphere, optical power, astigmatism, prism ) with an input device (instead of a passive determination by a sensor), or a continuous tuning of the parameters, wherein the user stops the tuning at the best value.

Preferably, according to a preferred embodiment of the invention, particularly method, the use of visual aids during the method (e.g. process of selecting said parameters) is explicitly excluded, wherein particularly the user is prompted to remove visual aids.

Further features and advantages of the present inventions as well as embodiments of the present invention shall be described in the following with reference to the Figures, wherein
- Fig. 1: an embodiment of a system according to the present invention for determining at least one optical property of an eyeglass for a person,
- Figs. 2A - 2C: different examples of test patterns that can be used in the system and method according to the present invention,
- Fig. 3: shows an embodiment of a lens shaping element of a tunable lens of the system,
- Fig. 4: shows a process of changing optical properties of a tunable lens of the system by deforming a surface of the tunable lens using an actuator on points distributed along a periphery of the shaping element,
- Fig. 5: shows a flow chart of an embodiment of the method according to the present invention,
- Fig. 6: shows an example of an interface of the system allowing a user to select different options of the system and method regarding eye training.
- Fig. 7: shows an embodiment of a display of the headset comprising light guides; and
- Fig. 8: shows a further embodiment of a display of the headset comprising light guides.

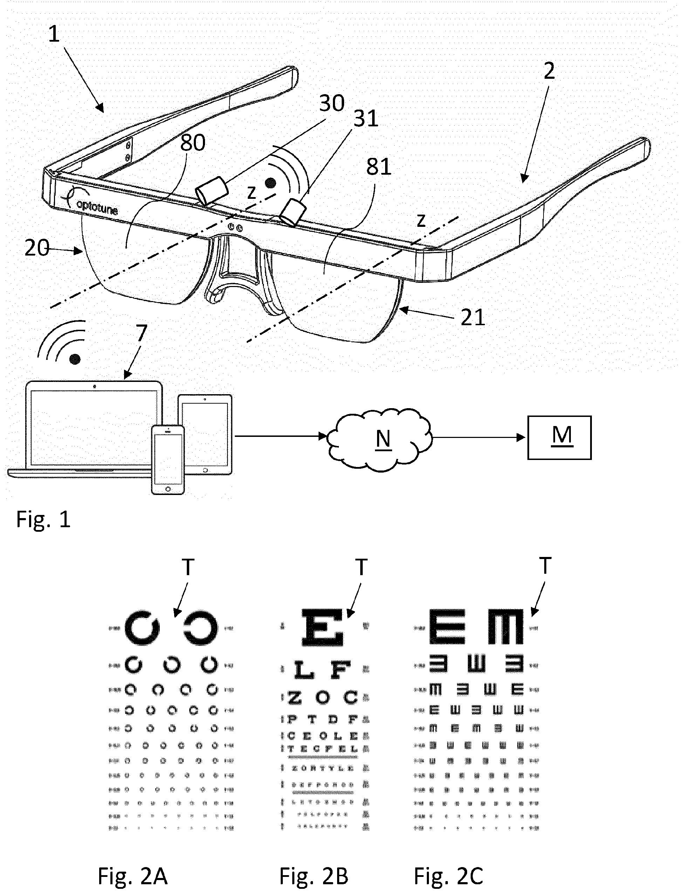

Fig. 1 shows an embodiment of a system 1 according to the present invention for determining at least one optical property of a visual aid for a person (e.g. of an eyeglass for a person). As indicated in Fig. 1, the system 1 comprises a VR and/or AR headset 2 configured to be worn by the person, the VR and/or AR headset 2 comprising at least one tunable lens 20 configured to be positioned in front of an eye of the person when the VR and/or AR headset is worn by the person. Preferably, the headset 2 comprises a further tunable lens 21 for the other eye of the person. Particularly, the first tunable lens 20 may be arranged in front of the right eye of the person while the further or second lens 21 may be arranged in front of the left eye of the person.

Particularly, the respective tunable lens 20, 21 is adjustable to adjust at least one optical property of the respective tunable lens 20, 21 so that the person perceives a test pattern T observed through the respective tunable lens 20, 21 as being sharp. Particularly, test patterns T such as shown in Figs. 2A to 2C can be used. Other suitable images may also be used. Particularly, the eyes can be examined separately, with the non-examined eye e.g. being closed, covered or otherwise separated from the optical path (e.g. by means of a shutter of the headset 2). Separate examination is particularly done for cylinder and sphere, while both eyes are considered in conjunction regarding prism.

Furthermore, the system 1 can comprise a sensor device 30, 31, the tunable lens(es) 20, 21 being configured to be adjusted to adjust a corresponding optical property of the respective lens 20, 21 until the sensor device 30, 31 detects accommodation of the respective eye of the person to the test pattern T observed through the respective tunable lens 20, 21. Particularly, the sensor device 30, 31 can comprise two cameras 30, 31, each camera 30, 31 being configured to monitor one eye of the person so that the left and right eye can be monitored separately. Particularly, accommodation of the respective eye can be detected by adjusting the corresponding optical property of the associated tunable lens 20, 21 in a continuous fashion (or in small successive increments) while observing the eye in questions of the person with the corresponding sensor device, particularly with the respective camera 30, 31, wherein the test pattern T is considered to be perceived as a sharp image by the person in case the lens of the eye under examination of the person stops changing its form at a certain value of the optical property. This can be inferred by automatically analyzing images of the lens of the eye acquired with the associated camera 30, 31. Such an analysis can be carried out by a computer 7 of the system 1 indicated in Fig. 1. The computer 7 can be any suitable computer such as a desktop computer, a laptop, a tablet or a smart phone (Fig. 1 depicts a selection of such computers although only one computer may be present in the system). It is also conceivable to have a computer 7 being integrated into the headset 2.

Furthermore, the headset 2 preferably comprises a display 80, 81 for each eye. In case of a VR headset 2, each display 80, 81 can be positioned in front of the respective eye when the VR headset 2 is worn for displaying information such as the test pattern T to the person. The respective tunable lens 20, 21 can then be arranged between the respective display 80, 81 and the respective eye. In case the headset 2 is an AR headset the respective display 80, 81 can be formed by the respective tunable lens 20, 21, i.e. by projecting information onto a surface of the respective tunable lens.

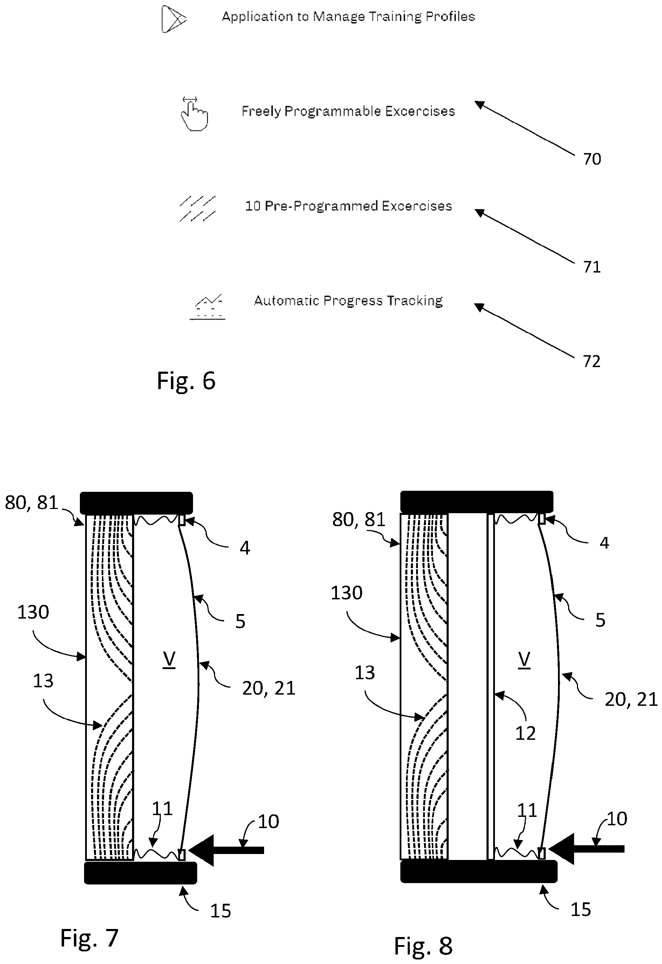

Particularly, as shown in Figs. 7 and 8, in case of an AR headset 2, the respective display 80, 81 can be transparent. Particularly, for an AR headset 2 the display 80, 81 can comprise a transparent carrier 130 comprising light guides 13, each light guide 13 representing a pixel of the display 80, 81 via which augmented reality can be presented to the person (in case of VR, the respective display does not need to be transparent). Further, as shown in Fig. 7, the surface of the tunable lens 20, 21 facing away from the person can be formed by the carrier 130 comprising the light guides 13. Alternatively, said surface can be formed by a separate transparent plate 12. A lens shaping element 4 can be connected to the carrier 130 (Fig. 7) or to the plate 12 (Fig. 8) via a bellows 11 or another suitable structure. The lens shaping element 4 can be changed in its shape and/or position using an actuator 10. Particularly, the respective tunable lens 20, 21 and carrier 13 and particularly plate 12 can be held by a frame 15. Further, in AR, the test pattern T can also be displayed via a separate display of the computer 7 or can be a physical object observed by the person through the tunable lens(es) 20, 21 of the headset 2.

The system 1 can be used to conduct the method according to the present invention for determining at least one optical property of a visual aid (e.g. eyeglass) for a person (e.g. so as to automatically generate an eyeglass prescription for the person) comprising the steps of:
a) Observation of the test pattern T by the person through a tunable lens 20, 21 of the headset 2 worn by the person, the tunable lens being 20, 21 positioned in front of an eye of the person, and
b) Adjusting at least one optical property of the tunable lens 20, 21 so that the person perceives the test pattern as being sharp
   or
   Adjusting at least one optical property of the tunable lens 20, 21 until the sensor device 30, 31 detects accommodation of the eye of the person to the test pattern T.

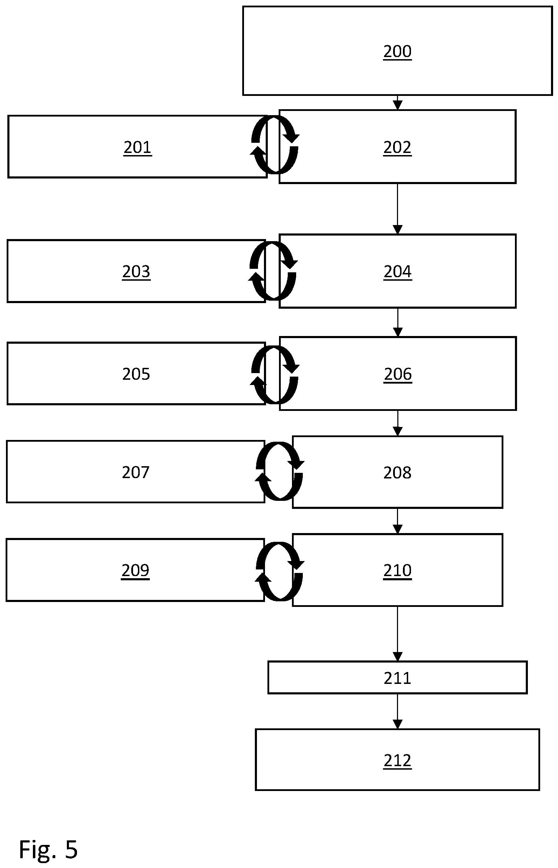

Particularly, according to an embodiment, a complete set of optical properties, as can be required by an eyeglass prescription, can be inferred by means of the embodiment of the method shown in Fig. 5.

According to a first step 200, the test pattern T (such as e.g. shown in Figs. 2A to 2C) is displayed by the VR and/or AR headset 2 or via an external display (e.g. of computer 7).

Thereafter, in step 201 the sphere power of the tunable lens 20 is tuned while it is checked 202 which value of the sphere power results in a best perception of the test pattern T. This optimal value of the sphere power is stored.

Thereafter, in step 203 the cylinder angle is tuned for a pre-defined small cylinder power while it is checked 204 which value of the cylinder angle results in a best perception of the test pattern T. This optimal value of the cylinder angle is stored.

Thereafter, in step 205 the cylinder power of the tunable lens 20 is tuned (with the cylinder angle assuming the optimal value) while it is checked 206 which value of the cylinder power results in a best perception of the test pattern T. This optimal value of the cylinder power is stored.

Thereafter, in step 207 the prism angle is tuned for a pre-defined small prism power (e.g. ± 0.5) while it is checked 208 which value of the prism angle results in a best perception of the test pattern T. This optimal value of the prism angle is stored.

Thereafter, in step 209 the prism power of the tunable lens 20 is tuned (with the prism angle assuming the optical value) while it is checked 210 which value of the prism power results in a best perception of the test pattern T. This optimal value of the prism power is stored.

In step 211 the visual aid/eyeglass prescription can be derived from the stored optimal values of the optical properties.

Finally, in step 212 the visual aid (e.g. eyeglasses) can be fabricated according to the measured optimal values of the optical properties (e.g. from the derived eyeglass prescription). The optimal values (e.g. eyeglass prescription) may be send to a manufacturer M, a manufacturing device M, or an optician using computer 7 via a computer network N such as the internet (e.g. via e-mail or via an upload to a website of a manufacturer). The optimal values or prescription may also be printed out and taken to a manufacturer or optician for obtaining the visual aid (e.g. eyeglasses).

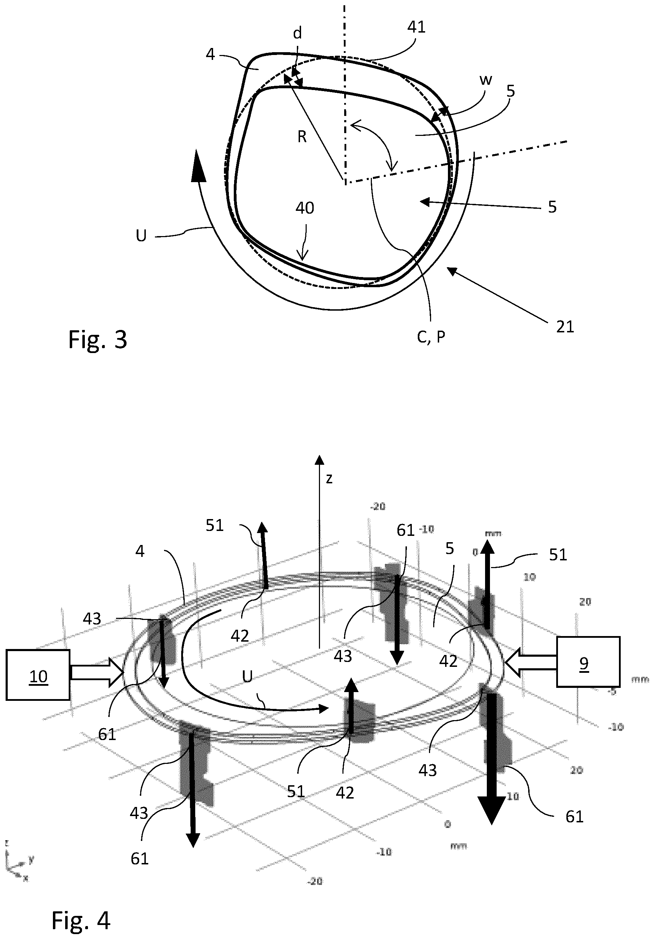

Furthermore, as indicated in Fig. 3, for adjusting the individual optical property of the respective tunable lens 21, here e.g. for the left eye of the person, so as to particularly give the lens 21 a desired sphere power, cylinder power, prism power, cylinder angle and prism angle), the respective tunable lens 21 can comprise a fluidic volume V, a flexible membrane 5 and a shaping element 4, wherein the membrane 5 delimits the fluidic volume V on one side, and wherein the shaping element 4 is attached to the membrane 5. Further, particularly, the shaping element 4 surrounds an optically active region of the membrane 5, wherein the shaping element 4 is arranged and configured to alter said at least one optical property of the tunable lens 20, 21 by being deflected by means of an actuator 10. This changes the shape of the surface of said active region of membrane 5 and therewith - depending on the deflection - the addressed optical properties (cf. also Figs. 7 and 8)

Particularly, Fig. 3 shows the shaping element 4 of the respective tunable lens 20, 21 in a top view along the optical axis z of the respective tunable lens 20, 21 (cf. also Fig. 1). The shaping element 4 preferably comprises a non-circular ring shape, and an inner edge of the shaping element 4 defines a non-circular contour 41.

Particularly, the shaping element 4 extends within an imaginary circumcircle 40. The circumcircle 41 is a circle surrounding the shaping element 4, in particular the contour 40, within the main extension plane of the shaping element 4, wherein the circumcircle 41 has the smallest radius possible. Particularly, a lateral distance d between the circumcircle 41 and the contour 40 varies along a perimeter U of the shaping element 4. The lateral distance d is measured along the radius R the circumcircle 41. The shaping element 4 can further have a width w which varies along the perimeter U of the shaping element 4. The width w is measured in a direction along the radius R of the circumcircle 41.

Particularly, by way of deflection of the lens shaping element 4, one of, a selection of or all of the following optical properties: sphere power, cylinder power, prism power, cylinder angle, prism angle, can be adjusted.

In this regard, sphere power (abbreviated as SPH) indicates the amount of lens power, measured in diopters of focal length. The deflection of the membrane for sphere is equal in all meridians of the tunable lens. The shaping element 4 is configured to alter the lens power by a definable deformation of the membrane.

Cylinder power (abbreviated as CYL) indicates the lens power for astigmatism of the tunable lens 21. The membrane 5 has a non-spherical surface shape for generating cylinder power. In particular, for generating cylinder power the membrane has a shape so that along a first meridian the membrane has no added curvature, and along a second meridian the membrane 5 has the maximum added curvature, wherein the first meridian and the second meridian extend perpendicular with respect to each other. The shaping element is deformable to alter the curvature of the membrane 5 along the second meridian.

Cylinder angle describes the angle of the first meridian, which has no added curvature to correct astigmatism. In other words, the cylinder angle is the angle of the first lens meridian that is 90 degrees away from the second meridian, wherein the second meridian contains the cylinder power. The cylinder angle is defined with an angle from 1° to 180°. The shaping element 4 is deformable to alter the cylinder axis from 1° to 180°angle.

Furthermore, prism power is the amount of prismatic power of the tunable lens 21, measured in prism diopters ("p.d." or a superscript triangle). Prism power is indicated in either metric or fractional English units (0.5 or ½, for example). Prism corresponds to a tilt of the membrane's 5 surface with respect to the optical axis z. Prism power defines the absolute of the angle by which the membrane's 5 surface is tilted. The shaping element 4 may be deformable to alter the prism power.

Prism angle is the direction of prismatic power of the tunable lens 21. The prism angle indicates the angle of the meridian around which the surface of the tunable lens is tilted with respect to the optical axis. The prism angle may extend along any meridian. The prism angle may be defined by an angle from 1° to 360°. The shaping element may be deformable to alter the prism angle from 1° to 360°.

Preferably, as indicated in the embodiment shown in Fig. 4, the tunable lens 21 comprises at least five actuation points, wherein at each actuation point there is a deflection point 42, a retention point 43 or both. Preferably, the tunable lens 21 comprises at least six actuation points, highly preferred at least eight actuation points. At the actuation points 42, 43 the deflection force 51 and/or the retention force 61 is transferred to the shaping element 4. In particular, at the actuation point 42, 43 the position of the shaping element 4 along the optical axis z is definable by the deflection force 51 and/or the retention force 61. For example, the actuation points 42, 43 are discrete points, wherein the shaping element 4 adapts its position along the optical axis z to the deflection of the neighboring actuation points 42, 43.

Particularly, at the retention points 43, the shaping element 4 can be connected to a mount 9 (such as a frame) via a rigid connection or via a spring, wherein an actuator 10 can be configured to exert a force at the respective actuation point along the optical axis z, wherein these forces can be independent from one another and can be individually adjusted. Particularly, the actuator can be an actuator system that comprise multiple actuators at each actuation point (e.g. based on shape memory alloys, piezo actuators etc.) to deflect the shaping element 4 at the respective actuation point by a predefined travel. in

Particularly, Fig. 4 shows the shaping element 4 in a specific tuned state in a schematic perspective view. The deflection points 42 and the retention points 43 are arranged along the perimeter U spaced apart from one another. Alternatively, at least some of the retention points 42 and some of the deflection points 41 may coincide. Furthermore, also shown is the deflection force 51 and the retention force 61 applied to the respective points 42, 43.

Finally, as indicated in Fig. 6, the system 1 according to the present invention may also be employed for eye training. Particularly, a user interface of computer 7 shown in Fig. 1 may be configured to display to the user a menu for selecting training options, such as "freely programmable exercises" 70, "Pre-programmed exercises" 71, and an "automatic progress tracking" 72 of the eye training performed by the person.

## Claims

1. A method for determining an optimal value of an optical property for a visual aid for a person, the method comprising the steps of:
a) providing a virtual reality (VR) and/or augmented reality (AR) headset comprising a tunable lens (20, 21),
b) Observation of a test pattern (T) by the person through the tunable lens (20, 21), and
c) Adjusting an optical property of the tunable lens (20, 21) to an optimal value, wherein an image of the test pattern perceived by the person is changed by adjusting said optical property

2. The method according to claim 1, wherein step c) further comprises that
- the person adjusts the optical property to an optimal value, or
- a sensor device (30, 31) detects an optimal value of the optical property.

3. The method according to claim 1 or 2, wherein adjusting the optical property comprises changing a shape of a surface of the tunable lens (20, 21) by means of an actuator.

4. The method according to one of the preceding claims, wherein the optical property of the tunable lens (20, 21) is one of: sphere power, cylinder power, cylinder angle (C), prism power, prism angle (P).

5. The method according to one of the preceding claims, wherein the optical property of the tunable lens is sphere power, and step c) further comprises
adjusting, as a further optical property, the cylinder angle of the tunable lens (20, 21) with the tunable lens comprising a predefined cylinder power until the optimal value of the cylinder angle is selected or detected.

6. The method according to claim 5, wherein step c) further comprises
adjusting, as a further optical property, the cylinder power of the tunable lens (20, 21) until the optimal value of the cylinder power is selected or detected, wherein the cylinder angle is at the selected or detected optimal value.

7. The method according to claim 6, wherein step c) further comprises
adjusting, as a further optical property, the prism angle of the tunable lens (20, 21) with the tunable lens comprising a predefined prism power until the optimal value of the prism angle is selected or detected.

8. The method according to claim 7, wherein step c) further comprises
adjusting, as a further optical property, the prism power of the tunable lens (20, 21) with the prism angle being set to the optimal value of the prism angle.

9. The method according to one of the preceding claims, wherein the method further comprises the step of
d) fabricating the visual aid, the visual aid comprising the optimal value of the respective optical property.

10. The method according to one of the claims 1 to 8, the method further comprising the step of:
d1) transmitting the optimal value of the respective optical property to a manufacturing device (M) for optical elements, and
d2) fabricating optical elements having the optimal value of the respective optical property, and particularly integrating the optical elements in a visual aid.

11. The method according to claim 9, wherein step d) comprises transmitting the optimal value of the respective optical property via a computer network (N).

12. A computer program comprising instructions which, when the computer program is executed on a computer (7) cause the computer to conduct the method according to one of the preceding claims.

13. A system (1) for determining at least one optical property of a visual aid for a person, the system comprising a VR and/or AR headset (2) configured to be worn by the person, the VR and/or AR headset (2) comprising a tunable lens (20, 21) configured to be positioned in front of an eye of the person when the VR and/or AR headset (2) is worn by the person, wherein the tunable lens (20, 21) is adjustable to adjust at least one optical property of the tunable lens (20, 21), and the system comprises an input device (7) configured to be operated bv the person to adjust the tunable lens and therewith the at least one optical property, and/or wherein the system (1) comprises a sensor device, wherein the sensor device is configured to detect accommodation of said eye and to provide data based on the detected accommodation, wherein the system is configured to control adjusting of the tunable lens and therewith of the property based on said data.

14. The system according to claim 13, wherein the system (1) comprises at least one display (80, 81), the display configured to display the test pattern, wherein particularly the at least one display is comprised by the VR and/or AR headset.

15. The system according to claim 13 or 14, wherein the system comprises an actuator (10) configured to change a shape of a surface of the tunable lens (20, 21) to adjust said at least one optical property.

16. The system according to one of the claims 13 to 15, wherein said at least one optical property is one of: sphere power, cylinder power, cylinder angle, prism power, prism angle.

17. The system according to one of the claims 13 to 16, wherein the system comprises a computer (7) configured to transmit the at least one optical property to a remote server via a computer network.
